# EUROPÄISCHE PATENTSCHRIFT

(11) **EP 0 866 060 B1**
(45) Veröffentlichungstag und Bekanntmachung des Hinweises auf die Patenterteilung: **20.11.2002**
(21) Anmeldenummer: 98102134.8
(22) Anmeldetag: 07.02.1998
(51) Int. Cl.: C07D 211/94

(54) **Verfahren zur Herstellung von 1,2,3,6-Tetrahydro-2,2,6,6,-tetramethylpyridin-N-oxyl**
Process for the preparation of 1,2,3,6-tetrahydro-2,2,6,6,-tetramethylpyridin-N-oxyl
Procédé pour la préparation de 1,2,3,6,-tetrahydro-2,2,6,6,-tetramethylpyridin-N-oxyl

(30) Priorität: 18.03.1997 DE 19711226
(43) Veröffentlichungstag der Anmeldung: 23.09.1998
(73) Patentinhaber: Degussa AG, 40474 Düsseldorf (DE)
(72) Erfinder: Kaufhold, Manfred, Dr., 45770 Marl (DE)

(56) Entgegenhaltungen:
- EP-A- 0 574 667
- DE-C- 283 864
- GB-A- 1 199 351
- ROZANTSEV E G ET AL: "SYNTHESIS AND REACTIONS OF STABLE NITROXYL RADICALS. I SYNTHESIS" SYNTHESIS, Nr. 4, 1. April 1971, Seiten 190-202, XP000568359
- D. BORDEAUX ET AL.: "Synthèse de radicaux libres nitroxydes dérivés du tétraméthyl-2,2,6,6 tétradydropyridine-1,2,3,6; structure de l'epoxy-3,4 tétramethyl-2,2,6,6 pipéridine-oxyle,C9H16NO2" ACTA CHRYSTALLOGR., SECT. C: CRYST. STRUCT. COMMUN., Bd. c39, Nr. 12, 1983, Seiten 1656-9, XP002074810

## Beschreibung

Die Erfindung betrifft ein Verfahren zur Herstellung von 1,2,3,6-Tetrahydro-2,2,6,6-tetramethylpyridin-N-oxyl (im folgenden als Dehydro-TEMPO = DH-TEMPO bezeichnet) durch katalytische Oxidation von 1,2,3,6-Tetrahydro-2,2,6,6-tetramethylpyridin (THTMP).

Synthesen von N-Oxylen durch Oxidation von entsprechenden sekundären Aminen sind aus der Literatur bekannt. Sie unterscheiden sich vor allem hinsichtlich des eingesetzten Oxidationsmittels. So führen R. Winter und R. Malherbe die Oxidation mit organischen Hydroperoxiden. z.B. tert.-Butylhydroperoxid, durch (siehe EP-A-0 157 738), verwenden also ein teures Oxidationsmittel und erhalten in mindestens stöchiometrischen Mengen tert.-Butanol als Kopplungsprodukt. Dieselben Nachteile bestehen auch bei den häufig als Oxidationsmittel empfohlenen Percarbonsäuren. Beispielsweise verwenden Chou et al. 3-Chlorperoxybenzoesäure als Oxidationsmittel (siehe J.Org.Chem.39 [1947], 2356, 2360).

Wesentlich günstiger ist dagegen die Verwendung von Wasserstoffperoxid als Oxidationsmittel, weil es preiswert ist und als Kopplungsprodukt nur Wasser entsteht. D.P.Young sowie Rozantsev et al. empfehlen Wasserstoffperoxid als Oxidationsmittel und ein Salz der Wolframsäure als Katalysator (siehe GB 1 199 351 und Tetrahedron 20 [1964], 131, 137). Nachteilig bei diesem Verfahren sind die extrem langen Reaktionszeiten von mehreren Tagen und das Problem der Entsorgung des Katalysators, da dieser aus Umweltschutzgründen nicht in das Abwasser gelangen darf. Auch bei Verwendung von Natriumcarbonat als Katalysator muß man zu lange Reaktionszeiten in Kauf nehmen (siehe Soviet physics Doklaay 261, 1, [1981], 103, 110). Durch Einsatz von Phosphorwolframsäure als Katalysator wird zwar die Reaktionszeit erheblich verkürzt, der Katalysator ist jedoch nur aufwendig herzustellen, und das Problem einer umweltgerechten Entsorgung bleibt bestehen (siehe Cf.R.Brière, H.Lemaire und A.Rassat, Bull. Soc.Chim.France **11** [1965], 3273).

Allen erwähnten Oxidationsverfahren ist gemeinsam, daß sie einen hohen Verbrauch an Chemikalien haben, technisch aufwendig sind, zu Problemen der Abfallbeseitigung führen und/oder unwirtschaftlich lange Reaktionszeiten erfordern.

Diese Nachteile werden durch das Verfahren nach EP 0 574 667 beseitigt, bei dem man 2,2,6,6-Tetramethylpiperidin mit Wasserstoffperoxid als Oxidationsmittel und Erdalkalimetallsalzen als Katalysatoren oxidiert.

Es wurde nun überraschend gefunden, daß sich auch das olefinisch ungesättigte 1,2,3,6-Tetrahydro-2,2,6,6-tetramethylpyridin-N-oxyl durch katalytische Oxidation von 1,2,3,6-Tetrahydro-2,2,6,6-tetramethylpyridin vorteilhaft herstellen läßt, wenn man 1,2,3,6-Tetrahydro-2,2,6,6-tetramethylpyridin in Anwesenheit eines Erdalkalimetallsalzes oder -hydroxids als Katalysator in wäßrigem Medium mit Wasserstoffperoxid oxidiert.

Die Reaktion wird durch das folgende Reaktionsschema wiedergegeben:

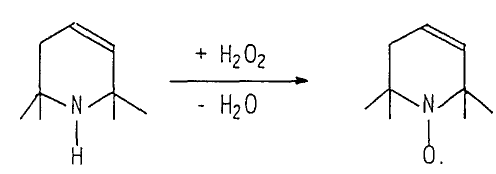

Bei dem erfindungsgemäßen Verfahren wird THTMP durch Wasserstoffperoxid unter Katalyse mittels Erdalkalimetallsalzen mit sehr hoher Selektivität von über 90% zum entsprechenden N-Oxyl, DH-TEMPO, oxidiert. Überraschenderweise wird die olefinische Doppelbindung weder im THTMP noch im DH-TEMPO angegriffen. Man mußte erwarten, daß diese Doppelbindung mit Wasserstoffperoxid reagieren würde, denn eben dies wird in der DE-OS 28 38 364 bei der Herstellung von 2,2,6,6-Tetramethylpiperidin-3,4-diol beschrieben. Bei dem erfindungsgemäßen Verfahren führen selbst Überschüsse von Wasserstoffperoxid nicht zu einer nennenswerten Reaktion der Doppelbindung.

DH-TEMPO ist ein hervorragender Polymerisationsinhibitor für Monomere, wie Acrylsäure und deren Ester, weil es polymerisationsinitiierende Radikale abfängt, auch in der Gasphase. Es zeigt eine für diese Verwendung günstige Kombination wünschenswerter Eigenschaften:
1. Es ist als Flüssigkeit mit niedrigem Erstarrungspunkt gut handhabbar. TEMPO hat dagegen einen Fp. von 35°C, ist also je nach Umgebungsbedingungen flüssig oder fest.
2. Es ist in Kohlenwasserstoffen, wie Toluol, gut löslich.
3. Es hat einen höheren Dampfdruck als die in 4-Stellung substituierten Derivate nach der EP 0 574 667, was für die Verwendung als Inhibitor in der Gasphase günstig ist.

Weiterhin eignet sich DH-TEMPO sehr gut als Redox-Katalysator.

Als Katalysatoren eignen sich Salze und Hydroxide des Calciums. Strontiums, Bariums und insbesondere des Magnesiums, z.B. die Chloride, Sulfate, Nitrate, Phosphate, Formiate, Acetate usw.. Voraussetzung ist, daß die Salze oder Hydroxide bei den Reaktionstemperaturen wenigstens teilweise im Reaktionsgemisch löslich sind und insoweit in dem Reaktionsgemisch weitgehend in Ionenform vorliegen. Die Salze oder Hydroxide können als Feststoff, als Gemisch von Feststoffen oder in Form einer Suspension oder Lösung eines Feststoffes oder mehrerer Feststoffe eingesetzt werden. Selbstverständlich können auch Erdalkalimetallverbindungen verwendet werden, die mit Bestandteilen des Reaktionsgemisches reagieren, z.B. hydrolysieren, und dann in Ionen dissoziieren, wie Erdalkalimetallalkoholate und Grignard-Verbindungen. Auch die Metalle selbst sind verwendbar, da sie unter den Reaktionsbedingungen mit Wasser reagieren.

Überraschend und vorteilhaft ist es, daß schon niedrige Konzentrationen an Erdalkalimetallsalz völlig ausreichen, um die hohen Ausbeuten des erfindungsgemäßen Verfahrens zu erzielen. Gute Ergebnisse werden bereits erzielt, wenn die Erdalkalimetallionen in 0,002-molarer Konzentration vorliegen. Zu den gut geeigneten Erdalkalimetallsalzen zählen Magnesiumsulfat-Heptahydrat, Magnesiumchlorid-Hexahydrat und Magnesiumnitrat-Hexahydrat. Das molare Verhältnis von THTMP zu Erdalkalimetallsalz bzw. -hydroxid beträgt in der Regel 10⁵:1 bis 10:1, vorzugsweise 10⁵:1 bis 10²:1 und insbesondere etwa 10⁴:1. Wegen dieser geringen Mengen ist der Katalysatorverbrauch gering. zudem sind die Erdalkalisalze und -hydroxide wohlfeile und unter Umweltaspekten unproblematische Stoffe. Insbesondere Verbindungen des Calciums und des Magnesiums sind in der Natur weit verbreitet, so daß geringe Mengen solcher Salze in den Abfallprodukten des Verfahrens die Umwelt nicht nennenswert belasten.

Die Umsetzung wird in wäßrigem Medium durchgeführt, d.h. in Wasser oder unter Mitverwendung eines mit Wasser mischbaren inerten organischen Lösungsmittels. THTPM bildet mit Wasser beim Rühren eine Emulsion, die in dieser Form mit Wasserstoffperoxid reagiert. Lösungsmittel erhöhen die Löslichkeit des THTMP; sie erhöhen im allgemeinen die Reaktionsgeschwindigkeit und verkürzen die Reaktionszeiten. Als Lösungsmittel werden aus wirtschaftlichen Gründen niedere Alkohole oder Diole verwendet, wie Methanol, Ethanol, n- oder iso-Propanol, Ethylenglykol oder Propylenglykol. Lösungsmittel mit einem niedrigen Dampfdruck werden aus Sicherheitsgründen bevorzugt, weil sie mit dem sauerstoffreichen Abgas keine explosiven Gemische bilden können. Bevorzugte Lösungsmittel sind hochsiedende Diole oder Diolether, wie Ethylenglykol, Propylenglykol, Ethylenglykolmono- oder -dialkylether, Propylenglykolmono- oder -dialkylether, Polyethylenglykole, Polyethylenglykolmono- oder -dialkylether, Polypropylenglykole sowie Polypropylenglykolmono- oder -dialkylether.

Wasserstoffperoxid kann in den handelsüblichen Formen als 10- bis 60 gew.-%ige Lösung eingesetzt werden.

THTMP wird aus dem entsprechenden gesättigten Alkohol; dem 4-Hydroxy-2,2,6,6-tetramethylpiperidin, durch Wasserabspaltung nach literaturbekannten Verfahren hergestellt (siehe E.Fischer, Ber. 16. 1604; Ber. 17, 1790).

Die praktische Durchführung des Verfahrens erfolgt z.B. wie folgt: THTMP, Katalysator, Wasser und gegebenenfalls ein Lösungsmittel werden vorgelegt, wobei die Gewichtsmenge des Wassers zweckmäßig das 0,1bis 2-fache, vorzugsweise das 0,3- bis 0,8-fache der Gewichtsmenge an THTMP beträgt. Die Gewichtsmenge des gegebenenfalls mitverwendeten Lösungsmittels beträgt zweckmäßig das 0,1 bis 2-fache der Gewichtsmenge des eingesetzten Wassers. Die Temperatur des vorgelegten Gemisches wird zweckmäßig auf 0 bis 100°C, bevorzugt auf 40 bis 90°C eingestellt. Innerhalb von 0,1 bis 2 Stunden wird das Wasserstoffperoxid zugegeben und das Reaktionsgemisch bei der gewählten Temperatur zweckmäßig noch 1 bis 30, vorzugsweise 5 bis 15 Stunden. weitergerührt. Alternativ kann man die Zugabe des Wasserstoffperoxids ein oder mehrere Male unterbrechen, um zunächst noch vorhandenes Wasserstoffperoxid aufzubrauchen. Das Molverhältnis von THTMP zu Wasserstoffperoxid beträgt zweckmäßig 1:1 bis 1:10, vorzugsweise 1:1 bis 1:5 und insbesondere 1:1,5 bis 1:2,5. Der Reaktionsverlauf kann durch gaschromatographische Analyse überwacht werden.

Die Aufarbeitung des Reaktionsgemisches erfolgt nach üblichen Methoden und ist in den meisten Fällen sehr einfach, da das THTMP fast vollständig umgesetzt wird. Beispielsweise wird nach einer Reaktion ohne organisches Lösungsmittel das Reaktionsgemisch einfach aufgearbeitet. indem das Wasser im Vakuum abdestilliert wird. Als Rückstand verbleibt DH-TEMPO mit einer gaschromatisch bestimmten Reinheit von über 95%. Da die Katalysatorkonzentration in der Regel kleiner als 1% ist, erübrigt sich für die meisten Anwendungen eine Abtrennung. Selbstverständlich kann das Reaktionsgemisch auch durch Extraktion aufgearbeitet und der Extrakt durch verschiedene Wäschen gereinigt werden. Diese Variante empfiehlt sich besonders, wenn ein hochsiedendes Lösungsmittel mitverwendet wurde. Für viele Zwecke ist der Extrakt ohne weitere Reinigung direkt einsetzbar, z.B. eine Lösung in Toluol.

Die folgenden Beispiele sollen die Erfindung näher erläutern, ohne ihren Anwendungsbereich einzugrenzen.

### Beispiel 1

Man benutzt eine Glasapparatur, die aus einem Dreihalskolben mit Rührer, Thermometer und Tropftrichter besteht, und setzt ein:

| | |
|---|---|
| 70,0g (0,5 Mol) | 1,2,3,6-Tetrahydro-2,2,6,6-tetramethylpyridin (= THTMP) |
| 50,0g | Wasser |
| 0,4g | Magnesiumsulfat-Heptahydrat |
| 72.9g | Wasserstoffperoxid (30Gew.-%) |

THTMP, Wasser und Magnesiumsulfat-Heptahydrat werden vorgelegt, und das Gemisch wird unter Rühren auf 70°C erwärmt. Innerhalb von 2 Stunden wird das Wasserstoffperoxid zugetropft. Danach rührt man 3 Stunden, setzt dann weiteres Wasserstoffperoxid, 27,0g, zu und läßt das Gemisch unter Rühren nachreagieren. Nach 7 Stunden Nachreaktion bei 70°C liegt der N-Oxyl-Gehalt bei ca. 80%. Durch zweimalige Zugabe von je 25,0 g weiterem Wasserstoffperoxid und Nachreaktion von jeweils 2 Stunden steigt der Gehalt an N-Oxyl auf 97.2%.

Zur Zersetzung von überschüssigem Wasserstoffperoxid wird das Reaktionsgemisch zunächst bei 80°C und dann bei 90°C jeweils eine halbe Stunde gerührt. Dann wird das Gemisch abgekühlt, und die Phasen werden getrennt. Es werden 71,0g organische Phase erhalten.

Zur Abtrennung von nicht umgesetztem THTMP wird die organische Phase mit 10%-iger Schwefelsäure gewaschen und mit festem Natriumbicarbonat neutral gestellt. Die Reinheit (GC-Analyse) beträgt 99%. Die Ausbeute errechnet sich zu 91,3 %d.Th., bezogen auf den Einsatz an THTMP. Der Erstarrungspunkt des flüssigen, rotgefärbten DH-TEMPO beträgt -10°C, es ist in jedem Verhältnis mit Toluol mischbar. Das Produkt hat die erwähnte Kombination günstiger Eigenschaften.

### Beispiele 2 bis 4

Man verwendet die im Beispiel 1 beschriebene Apparatur, setzt die dort genannten Mengen an THTMP, Wasser und Wasserstoffperoxid ein und gibt als Katalysator jeweils 0,02 Mol folgender Magnesiumverbindungen zu:
Magnesiumhydroxid
Magnesiumchlorid
Magnesiumnitrat

Man verfährt, wie in Beispiel 1 beschrieben, und erhält sehr ähnliche Ergebnisse.

### Beispiel 5

Man benutzt die im Beispiel 1 beschriebene Apparatur, setzt die dort genannten Mengen an THTMP und Magnesiumsulfat-Heptahydrat ein und gibt an Stelle des reinen Wassers ein Gemisch aus 30,0g Polyethylenglykol (Molgewicht ca. 2.000) und 20g Wasser hinzu. Die Reaktion verläuft erheblich schneller und ist nach 7 Stunden beendet. Der Verbrauch an Wasserstoffperoxid ist mit 95g erheblich geringer als im Beispiel 1. Die Aufarbeitung erfolgt durch Extraktion mit Toluol. die Ausbeute beträgt 92,5%.

## Patentansprüche

1. Verfahren zur Herstellung von 1,2,3,6-Tetrahydro-2,2,6,6-tetramethylpyridin-N-oxyl durch katalytische Oxidation von 1,2,3,6-Tetrahydro-2,2,6,6-tetramethylpyridin **dadurch gekennzeichnet, daß** man 1,2,3,6-Tetrahydro-2,2,6,6-tetramethylpyridin in Anwesenheit eines Erdalkalimetallsalzes oder -hydroxids als Katalysator in wäßrigem Medium mit Wasserstoffperoxid oxidiert.

2. Verfahren nach Anspruch 1, **dadurch gekennzeichnet daß** man als Katalysator die Erdalkalimetallsalze als Chloride, Sulfate, Nitrate oder Phosphate einsetzt.

3. Verfahren nach Anspruch 1 oder 2, **dadurch gekennzeichnet, daß** als Katalysatoren Magnesiumhydroxid oder Magnesiumsalze eingesetzt werden.

4. Verfahren nach Anspruch 3, **dadurch gekennzeichnet, daß** als Magnesiumsalze Magnesiumsulfat-Heptahydrat, Magnesiumchlorid-Hexahydrat und/oder Magnesiumnitrat-Hexahydrat eingesetzt werden.

5. Verfahren nach einem der Ansprüche 1 bis 4, **dadurch gekennzeichnet, daß** das molare Verhältnis von 1,2,3,6-Tetrahydro-2,2,6,6-tetramethylpyridin zum Eralkalihydroxid oder Erdalkalisalz 10⁵:1 bis 10:1, insbesondere 10⁵:1 bis 10²:1 beträgt.

6. Verfahren nach einem der Ansprüche 1 bis 5, **dadurch gekennzeichnet, daß** die Umsetzung in wäßriger Emulsion erfolgt.

7. Verfahren nach einem der Ansprüche 1 bis 5, **dadurch gekennzeichnet, daß** die Umsetzung unter Zusatz eines organischen Lösungsmittels, insbesondere eines hochsiedenden Diols oder Biolethers, erfolgt.

8. Verfahren nach Anspruch 7, **dadurch gekennzeichnet, daß** die Umsetzung unter Zusatz von Ethylenglykol, Propylenglykol, eines Ethylenglykolmono- oder -dialkylethers, eines Propylenglykolmono- oder -dialkylethers, von Polyethylenglykol, eines Polyethylenglykolmonooder -dialkylethers, von Polypropylenglykol oder eines Polypropylenglykolmono- oder -dialkylethers erfolgt.

9. Verfahren nach einem der Ansprüche 1 bis 8, **dadurch gekennzeichnet, daß** die Umsetzung im Temperaturbereich von 0 bis 100°C, insbesondere von 40 bis 90°C erfolgt.

## Claims

1. A process for the preparation of 1,2,3,6-tetrahydro-2,2,6,6-tetramethylpyridine N-oxide by the catalytic oxidation of 1,2,3,6-tetrahydro-2,2,6,6-tetramethylpyridine, **characterized in that** 1,2,3,6-tetrahydro-2,2,6,6-tetramethylpyridine is oxidized with hydrogen peroxide in an aqueous medium in the presence of an alkaline earth metal salt or hydroxide as catalyst.

2. A process according to claim 1, **characterized in that** the alkaline earth metal salts used as catalysts are the chlorides, sulphates, nitrates or phosphates.

3. A process according to claim 1 or 2, **characterized in that** magnesium hydroxide or magnesium salts are used as catalysts.

4. A process according to claim 3, **characterized in that** the magnesium salts used are magnesium sulphate heptahydrate, magnesium chloride hexahydrate and/or magnesium nitrate hexahydrate.

5. A process according to any one of claims 1 to 4, **characterized in that** the molar ratio of 1,2,3,6-tetrahydro-2,2,6,6-tetramethylpyridine to alkaline earth metal hydroxide or alkaline earth metal salt is 10⁵:1 to 10:1, especially 10⁵:1 to 10²:1.

6. A process according to any one of claims 1 to 5, **characterized in that** the reaction is carried out in aqueous emulsion.

7. A process according to any one of claims 1 to 5, **characterized in that** the reaction is carried out with the addition of an organic solvent, especially a high-boiling diol or diol ether.

8. A process according to claim 7, **characterized in that** the reaction is carried out with the addition of ethylene glycol, propylene glycol, an ethylene glycol monoalkyl or dialkyl ether, a propylene glycol monoalkyl or dialkyl ether, polyethylene glycol, a polyethylene glycol monoalkyl or dialkyl ether, polypropylene glycol or a polypropylene glycol monoalkyl or dialkyl ether.

9. A process according to any one of claims 1 to 8, **characterized in that** the reaction is carried out in the temperature range 0 to 100°C, especially 40 to 90°C.

## Revendications

1. Procédé de préparation de 1,2,3,6-tétrahydra 2,2,6,6-tétraméthylpyridine N-oxyl par oxydation catalytique de 1,2,3,6-tétrahydro 2,2,6,6,-tétraméthylpyridine,
**caractérisé en ce qu'**
on oxyde la 1,2,3,6-tétrahydro 2,2,6,6,-tétraméthylpyridine avec le peroxyde d'hydrogène en présence d'un sel de métal alcalinoterreux ou d'un hydroxyde de métal alcalinoterreux comme catalyseur en milieu aqueux.

2. Procédé selon la revendication 1,
**caractérisé en ce qu'**
on met en oeuvre comme catalyseur les sels de métal alcalinoterreux sous forme de chlorures, de sulfates, de nitrates ou de phosphates.

3. Procédé selon la revendication 1 ou 2,
**caractérisé en ce qu'**
on met en oeuvre comme catalyseurs, de l'hydroxyde de magnésium ou des sels de magnésium.

4. Procédé selon la revendication 3,
**caractérisé en ce qu'**
on met en oeuvre comme sels de magnésium l'heptahydrate de sulfate de magnésium, l'hexahydrate de chlorure de magnésium, et/ou l'hydrate de nitrate de magnésium.

5. Procédé selon l'une des revendications 1 à 4,
**caractérisé en ce que**
le rapport molaire de la 1,2,3,6-tétrahydro 2,2,6,6-tétraméthylpyridine, à l'hydroxyde de métal alcalin s'élève à 10⁵ : 1 à 10 : 1, en particulier de 10⁵: 1 à 10² : 1.

6. Procédé selon l'une des revendications 1 à 5,
**caractérisé en ce que**
la réaction s'effectue en émulsion aqueuse.

7. Procédé selon l'une des revendications 1 à 5,
**caractérisé en ce que**
la réaction s'effectue en ajoutant un solvant organique, en particulier un diol à point d'ébullition élevé ou un éther de diol.

8. Procédé selon la revendication 7,
**caractérisé en ce que**
la réaction s'effectue en ajoutant de l'éthylène glycol, du propylèneglycol, un mono- ou dialkyléther d'éthylèneglycol, un mono- ou un dialkyléther de propylèneglycol, un polyéthylèneglycol, un mono- ou un dialkyléther de polyéthylèneglycol, un polypropylèneglycol ou un mono- ou un dialkyléther de polypropylèneglycol.

9. Procédé selon l'une des revendications 1 à 8,
**caractérisé en ce que**
la réaction s'effectue dans la plage de températures allant de 0 à 100°C, en particulier de 40 à 90°C.
